(19) **Europäisches Patentamt / European Patent Office / Office européen des brevets**

(11) **EP 1 893 731 B1**

(12) **EUROPEAN PATENT SPECIFICATION**

(45) Date of publication and mention
of the grant of the patent:
**10.08.2011 Bulletin 2011/32**

(21) Application number: **06763775.1**

(22) Date of filing: **19.06.2006**

(51) Int Cl.:
***C11B 1/10*** (2006.01)   ***C12P 7/64*** (2006.01)

(86) International application number:
**PCT/EP2006/063317**

(87) International publication number:
**WO 2006/136539 (28.12.2006 Gazette 2006/52)**

(54) **PROCESS FOR OBTAINING LIPID FROM CELLS**

VERFAHREN ZUM ERHALT VON LIPID AUS ZELLEN

TRAITEMENT POUR OBTENIR UN LIPIDE À PARTIR DE CELLULES

(84) Designated Contracting States:
**AT BE BG CH CY CZ DE DK EE ES FI FR GB GR HU IE IS IT LI LT LU LV MC NL PL PT RO SE SI SK TR**

(30) Priority: **23.06.2005 EP 05105622**
**23.06.2005 US 693073 P**

(43) Date of publication of application:
**05.03.2008 Bulletin 2008/10**

(60) Divisional application:
**10183531.2**

(73) Proprietors:
• **DSM IP Assets B.V.**
**6411 TE Heerlen (NL)**
• **MARTEK BIOSCIENCES CORPORATION**
**Columbia, MD 21045 (US)**

(72) Inventors:
• **BIJL, Hendrik, Louis**
**NL-3131 ZD Vlaardingen (NL)**
• **VERKOEIJEN, Daniel**
**NL-2497 AW Den Haag (NL)**
• **NIGGLI, Heinz**
**Randolph, NY 07869 (US)**

(74) Representative: **Elkenbracht, Johan Christiaan et al**
**DSM Intellectual Property**
**P.O. Box 130**
**6100 AC Echt (NL)**

(56) References cited:
**WO-A-00/77183   WO-A-97/04121**
**WO-A-97/36996**

**Description**

The present invention relates to a process for obtaining lipid from a composition comprising cells and water.

Lipids can be produced by micro-organisms, for instance in a fermentation process. They can also be present in plant cells. Lipids have many uses, and can for example be included into foodstuffs, feed, nutritional supplements and pharmaceuticals.

Obtaining lipid from cells often involves obtaining the lipid from a composition comprising the cells and water. The composition can for instance be a fermentation broth or a partly dewatered product obtained by separating part of the water from a fermentation broth, for instance by mechanical separation techniques. Various processes are known to allow recovery of lipid from a composition comprising cells and water. For instance, it is known to dry the composition by spray drying, and to recover the lipid from the dried product by extraction with an organic solvent. However, the quality of the lipid can be impaired under the conditions that prevail during spray drying. Moreover, the morphology of the dried product may render subsequent extraction less efficient.

WO-A-97/36996 describes a process for obtaining lipid from microbial cells, which involves granulation of the cells into granules, preferably by extrusion. The granules are dried, and the lipid is obtained from the dried granules, for instance by extraction with an organic solvent. The extraction is efficient, the resulting lipid has a high quality, and the yield, i.e. the quantity of lipid recovered per quantity of lipid present in cells is high.

In spite of the advantages of the process of WO-A-97/36996, there is still a desire for further improvement. In particular, the process involves relatively many steps, such as granulation and drying of the granules. Moreover, the drying step requires energy. Therefore, there is a desire for a process requiring fewer steps, wherein energy can be saved, and which enables the lipid to be extracted in an efficient manner.

This goal is achieved according to the invention by providing a process for obtaining lipid from a composition comprising cells and water, said process comprising:

(a) contacting the composition with a desiccant; and
(b) recovering the lipid from the cells.

The process according to the invention enables lipid of high quality to be obtained with a high yield. According to the invention, drying the cells by heating prior to recovering the lipid from the cells is not needed. This is of particular advantage for heat sensitive lipid, for instance lipid comprising a polyunsaturated fatty acid (PUFA). The process according to the invention further has the advantage that formation of fines or dust - which are for instance formed by prior art processes that involve spray drying or milling - is avoided or minimized. Moreover, it is found that - when the lipid is recovered by extraction with a solvent - relatively small quantities of solvent suffice to obtain a relatively high yield. The invention also enables recovering the lipid by extraction in the presence of the desiccant, i.e. without the need of separating the desiccant from the cells. Hence, the extraction can be carried out in the presence of water adsorbed to or absorbed by the desiccant. This is surprising in that the prior art advocate to remove water in order to obtain high yields (see e.g. WO-A-97/36996.

The process according to the invention comprises contacting the composition comprising cells and water with a desiccant. As a result, water is taken up from the composition by the desiccant. Taking up water may be by adsorption and/or absorption. The desiccant is not limited to a specific desiccant, and a wide variety of agents capable of taking up water from the composition by adsorption and/or absorption may be used as desiccant. Such agents are also referred to in the art as adsorbents and/or absorbents.

Examples of desiccants that may be used in the process are for instance silica (e.g. silica gel), activated carbon, activated alumina, a molecular sieve (e.g. a carbon molecular sieve or a zeolite), or a cross-linked polymer (e.g a poly-acrylate). Silica is preferred. Preferably, the desiccant is in particulate form.

Preferably, the desiccant has a high absorption capacity. The absorption capacity refers to the quantity of water that can be absorbed per quantity of desiccant. A relatively high absorption capacity has the advantage that optimal extraction yields can be obtained using relatively small quantities of desiccant. The desiccant may have an absorption capacity of at least 50 g of water per 100 g of desiccant, preferably at least 100 g of water per 100 g of desiccant. There is no specific upper limit for the absorption capacity. In practice, the absorption capacity for water is below 20.000 g of water per 100 g of desiccant, for instance below 10.000 g of water per 100 g of desiccant. As used herein the weight of the desiccant refers to the moisture-free weight of the desiccant. The absorption capacity may be determined by techniques known to the skilled person. A suitable method is by DBP absorption, for instance according to DIN 53601.

Preferably, the desiccant has a high specific surface area. A relatively high specific surface area has the advantage that optimal extraction yields can be obtained using relatively small quantities of desiccant. The desiccant may for instance have a specific surface area of at least 25 m$^2$/g, preferably at least 50 m$^2$/g, preferably at least 100 m$^2$/g. There is no specific upper limit for the specific surface area of the desiccant. In practice, the specific surface area is below 2000 m$^2$/g, for instance below 1000 m$^2$/g. The specific surface area may be determined by techniques known to the skilled person, for instance according to ISO 5794/1, Annex D.

In a preferred embodiment, the desiccant is porous. This has the advantage that the surface area available for adsorption is relatively high.

In the process according to the invention, the composition to be contacted with the desiccant may comprise water in any suitable quantity. The water content of the composition to be contacted with the desiccant may for instance be at least 5 wt.%, for instance at least 10 wt.%, for instance at least 20 wt.%, for instance at least 30 wt.%. There is no specific upper limit for the water content of the composition to be contacted with the desiccant. The water content of the composition to be contacted with the desiccant may for instance be below 98 wt.%, preferably below 95 wt.%, preferably below 90 wt.%, preferably below 80 wt.%, preferably below 70 wt.%. A lower water content has the advantage that less desiccant may be used to obtain an optimal yield.

As used herein, the water content of the cells is given by $w_w/(w_{cells}+w_w)*100\%$ wherein

$w_w$ = weight of the water in the composition
$W_{cells}$ = weight of dry matter of the cells in the composition

The skilled man will understand that the weight of dry matter of the cells (cells) refers to the weight of the moisture-free cells, but including the weight of the lipid. It will be appreciated that the weight of the water comprised in the composition ($w_w$) includes the total weight of water in the composition, including intracellular, intercellular and extracellular water. The values of $W_w$ and $w_{cells}$, and accordingly the water content of the composition can be determined by methods known to the skilled person, for instance by evaporating the water at a temperature of 105 °C, and determining the weight of the evaporated water and the remaining cells. An infrared dry-matter balance may for instance be used.

In a preferred embodiment, the invention provides preferred quantities of desiccant to be contacted with the composition. It is found that the extraction yield increases with increasing quantity of desiccant that is contacted with the composition until an optimal yield is achieved. Based on this teaching provided by the invention, the skilled man can - by varying the quantity of desiccant - determine the optimal quantity of desiccant in any situation.

It is in particular found that the preferred quantities of desiccant are dependent on the water content of the composition and on the properties of the desiccant. In a preferred embodiment, $w_{des}/w_{cells(a)} + ww(a)) > (1/x)*(1- w_{cells(a)}/(0.8*(w_{cells(a)} + w_{w(a)})))$, wherein

$w_{des}$ = weight of desiccant to be contacted with the composition in (a)
$w_{cells(a)}$ = weight of dry matter of the cells in the composition to be contacted with the desiccant in (a)
$w_{w(a)}$ = weight of water in the composition to be contacted with the desiccant in (a)
$x$ = absorption capacity of the desiccant.

In another preferred embodiment,

$$w_{des}/(w_{cells(a)} + w_{w(a)}) > (1/x)*(1 - w_{cells(a)}/(0.9*(w_{cells(a)} + w_{w(a)})))$$

In a preferred embodiment, the process according to the invention comprises contacting the composition with a quantity of desiccant such that the yield is at least 70%, preferably at least 80%, more preferably at least 85%, more preferably at least 90%. As used herein, the yield refers to the quantity of lipid recovered from the cells per quantity of lipid present in the cells.

The composition to be contacted with the desiccant may be any suitable composition comprising cells and water from which lipid can be obtained. The composition may for instance be a fermentation broth, or a partly dewatered product obtained by separating of water, for instance by mechanical separation, from a fermentation broth. The composition may for instance be a wet cake, a sludge, slurry, concentrate and/or cream. Although granulation is not needed according to the invention, the process according to the invention also offers advantages when the cells are in the form of a granulate, for instance an extrudate.

Contacting the composition with the desiccant may be effected in any suitable manner, preferably by mixing the composition with the desiccant to obtain a mixture comprising the cells and the desiccant. The lipid may be recovered from the mixture, for instance by extraction with a solvent.

In view of the above, in a preferred embodiment, the invention provides a process for obtaining lipid from a composition comprising cells and water, said process comprising (a) mixing the composition with a desiccant to give a mixture comprising the cells and the desiccant; and (b) recovering the lipid from the mixture.

In another aspect, the invention also provides a mixture comprising (i) cells comprising a lipid and (ii) desiccant, as according to claim 27. It will be appreciated that preferred aspects and/or embodiments of the process according to the invention apply to the mixture according to the invention. A mixture of plant cells, phospholipids and a dessicant is described in WO-A-00/77183.

In a preferred embodiment, the mixture further comprising water, wherein

$$w_{des, \, mix}/(w_{cells, \, mix} + w_{w, \, mix}) > (1/x)^*(1 - w_{cells, \, mix}/(0.8^*(w_{cells, \, mix} + w_{w, \, mix}))),$$

preferably

$$w_{des, \, mix}/(w_{cells, \, mix} + w_{w, \, mix}) > (1/x)^*(1 - w_{cells, \, mix}/(0.9^*(w_{cells, \, mix} + w_{w, \, mix}))),$$

wherein

| | |
|---|---|
| $w_{des, \, mix}$ | = weight of the desiccant in the mixture |
| $w_{cells \, mix}$ | = weight of dry matter of the cells in the mixture. |
| $w_{w, \, mix}$ | = weight of the water in mixture |
| $x$ | = absorption capacity of the desiccant. |

Mixing can be carried out in any suitable mixer, for instance in a high shear mixer, for instance an Orbit Screw Mixer, a Plow Mixer, a Paddle Mixer, a Tumble Blender with intensifier bars or a Diosna Mixer.

The lipid may be recovered from the cells resulting from the contacting in (a) in any suitable manner. The lipid may for instance be recovered from the cells, for instance by extraction with a solvent, after or during contacting the composition with the desiccant.

In a preferred embodiment, the lipid is recovered from the cells by extraction with a solvent. A wide variety of solvents may be used. The solvent may for instance be a $c_{1-10}$ alkyl ester (e.g. ethyl or butyl acetate), toluene, a $C_{1-3}$ alcohol (e.g. methanol, propanol), a $C_{3-6}$ alkanes (e.g. hexane) or a supercritical fluid (e.g. liquid $CO_2$ or supercritical propane). Mixtures of the solvents are also possible. In a preferred embodiment, the solvent is an organic solvent. The process according to the invention is especially advantageous, if the solvent is an apolar solvent. Most preferably, the solvent is hexane or supercritical $CO_2$.

The cells or mixture comprising the cells may be contacted with the solvent in any suitable manner, for instance by percolation extraction, countercurrent extraction, or extraction in a mixer. Extraction in a mixer is preferred. In a preferred embodiment, between 2 to 40 weight parts of solvent are used per weight part of cells.

In an embodiment of the invention, at least part of the desiccant (e.g. at least 50%) is separated from the mixture prior to contacting the cells with the solvent. In a preferred embodiment, the mixture comprising the cells and at least part of the desiccant is contacted with the solvent. Accordingly, recovering the lipid by extraction with a solvent may be effected in the presence of (at least part of) desiccant. This has the advantage that a separation step to separate the desiccant from the cells is not needed. Surprisingly, the process according to the invention still results in an efficient extraction, even in the presence of water. In an embodiment,

$$w_{w(b)}/w_{cells(b)} > 0.5^*w_{w(a),}/w_{cells(a)}$$

wherein

| | |
|---|---|
| $w_{w(b)}$ | = weight of water in mixture from which the lipid is recovered in (b) |
| $w_{cells(b)}$ | = weight of dry matter of the cells in mixture from which the lipid is recovered in (b) |
| $w_{w(a)}$ | = weight of water in the composition to be contacted with the desiccant in (a) $w_{cells}(a)$ = weight of dry matter of the cells in the composition to be contacted with the desiccant in (a) |

In another embodiment,

$$w_{w(b)}/w_{cells(b)} > 0.7 {}^* w_{w(a)}/w_{cells(a)}$$

In another embodiment,

$$w_{w(b)}/w_{cells(b)} > 0.8 {}^* w_{w(a)}/w_{cells(a)}$$

In another embodiment,

$$w_{w(b)}/w_{cells(b)} > 0.9 {}^* w_{w(a)}/w_{cells(a)}$$

The process according to the invention may be used to obtain a wide variety of lipids from a wide variety of cells. The lipid may for instance comprise one or more of the following compounds: lipstatin, statin, TAPS, pimaricine, nystatine, fat-soluble antibiotic (e.g. laidlomycin) fat-soluble anti-oxidant (e.g. co-enzyme Q10), cholesterol, phytosterol, desmosterol, tocotrienol, tocopherol, carotenoid, or xanthophylls, for instance beta-carotene, lutein, lycopene, astaxanthin, zeaxanthin, or canthaxanthin, fatty acids, such as conjugated linoleic acids or polyunsaturated fatty acids (PUFAs). In a preferred embodiment, the lipid comprises at least one of the compounds mentioned above at a concentration of at 5 wt.%, more preferably at least 10 wt.% (with respect to the weight of the lipid).

Lipid may be obtained comprising for example triglyceride, phospholipid, free fatty acid, fatty acid ester (e.g. methyl or ethyl ester) and/or combinations thereof. In a preferred embodiment, the lipid has a triglyceride content of at least 50%, more preferably at least 70%, optimally at least 90%.

In a particularly preferred embodiment of the invention, the lipid comprises a polyunsaturated fatty acid (PUFA), for instance a PUFA having at least 18 carbon atoms, for instance a C18, C20 or C22 PUFA. In a preferred embodiment, the PUFA is an omega-3 PUFA ($\Omega$3) or an omega-6 PUFA ($\Omega$6). Preferably, the PUFA has at least 3 double bonds. Preferred PUFAs are:

docosahexaenoic acid (DHA, 22:6 $\Omega$3);
$\gamma$-linolenic acid (GLA, 18:3 $\Omega$6);
$\alpha$-linolenic acid (ALA, 18:3 $\Omega$3);
dihomo-$\gamma$-linolenic acid (DGLA, 20:3 Q6);
arachidonic acid (ARA, 20:4 $\Omega$6); and
eicosapentaenoic acid (EPA, 20:5 $\Omega$3).

In a preferred embodiment, the lipid comprises at least one PUFA (for instance ARA or DHA) at a concentration of at least 5 wt.%, for instance at least 10 wt.%, for instance at least 20 wt.% (with respect to the weight of the lipid).

The PUFA may be in the form of a (mono-, di, or tri) glyceride, phospholipid, free fatty acid, fatty acid ester (e.g. methyl or ethyl ester) and/or combinations thereof. Preferably, a lipid is obtained wherein at least 50% of all PUFAs is in triglyceride form.

The lipid may be an oil or fat, for instance an oil comprising a PUFA. Preferred embodiments for the lipid apply to the oil or fat mutatis mutandis.

The cells may be any cells comprising a lipid. Typically, the cells have produced the lipid. The cells may be whole cells or ruptured cells. The cells may be of any suitable origin. The cells may for instance be plant cells, for instance cells from seeds or cells of a micro-organism (microbial cells). Examples of, microbial cells are yeast cell, bacterial cells, fungal cells, and algal cells. Fungi are preferred, preferably of the order *Mucorales,* for example *Mortierella, Phycomyces, Blakeslea, Aspergillus, Thraustochytrium, Pythium or Entomophthora.* The preferred source of arachidonic acid (ARA) is from *Mortierella alpina, Blakeslea trispora, Aspergillus terreus or Pythium insidiosum.* Algae can be dinoflagellate and/or include *Porphyridium, Nitszchia, or Crypthecodinium (e.g. Crypthecodinium cohnii).* Yeasts include those of the genus *Pichia or Saccharomyces, such as Pichia ciferii.* Bacteria can be of the genus *Propionibacterium.* Examples of plant cells comprising a lipid are cells from soy bean, rape seed, canola, sunflower, coconut, flax and palm seed. In an embodiment of the invention, the cells are plant cells comprising lipid which lipid comprises ARA.

In an embodiment of the invention, the cells are produced by fermentation. Suitable processes for fermentation are known to the skilled person, and are for instance disclosed in WO-A-9737032. Preferably, the cells produced by fermentation are heated or pasteurised.

In a preferred embodiment, the process according to the invention comprises one or more of the following steps prior to contacting the composition with the desiccant: (i) heating or pasteurising the cells; (ii) separating water from the cells by mechanical separation; (iii) washing the cells; and (iv) squeezing the cells.

Heating or pasteurizing may be effected at a temperature of from 65 to 120°C. It may inactivate or denature enzymes such as lipases and/or lipoxygenases.

Separating water from the cells by mechanical separation can advantageously be used to obtain the preferred values for the water content and/or dry matter content as disclosed hereinabove. Mechanical separation may for instance involve filtering, centrifuging, squeezing, sedimentation, or the use of a hydrocyclone.

The lipid may further be treated in any suitable manner. If the lipid is recovered by extraction with a solvent, the lipid may be obtained from the solvent by evaporation of the solvent.

The lipid obtained or obtainable by the process according to the invention may be subjected to further treatments, for instance to acid treatment (also referred to as degumming), alkali treatment (also referred to as neutralization), bleaching, deodorizing, cooling (also referred to as winterization).

The lipid obtained or obtainable by the process according to the invention has many uses. It may for instance be used for the preparation of a food product, for instance a human food product (e.g. infant formula), or an animal feed product. It may also be used for the preparation of a pharmaceutical product or a cosmetic product.

The invention will now be elucidated with reference to the following examples, without however being limited thereto.

## Examples

### Comparative experiment A

A fermentation broth containing ARA was obtained by *Mortierella alpina* fermentation under the conditions as disclosed in WO-A-9736996, page 45, and was subsequently pasteurized at 65 °C for one hour. The broth was filtered using a belt filter, washed with water to remove residual medium components, and squeezed. The water content of the squeezed filter cake was 60.7 wt.%. The dry matter content (cells, including oil in the cells) was 39.3 wt.%. The squeezed filter cake was extruded as described in WO-A-9736996, followed by drying to a dry matter content of 94 wt.% using a fluid bed dryer.

Extraction was performed as follows. In a first extraction stage 100 grams of the dried product obtained as described above was added to 300 ml of laboratory-grade n-hexane, and magnetically stirred at room temperature (20 °C) for one hour. After the first extraction stage the liquid phase (comprising oil dissolved in hexane) was decanted from the solids (comprising cells). In a second extraction stage the remaining solids were added to 250 ml of n-hexane and stirred for 30 minutes. After the second extraction stage the liquid phase (comprising oil dissolved in hexane) was separated from the solids by vacuum filtration. The liquid phase from the first and second extraction stage were combined, after which the hexane was evaporated using a Rotavapor® obtained from Büchi, Switzerland. Operated at 68°C, and a pressure between 100 and 400 mbara for 30 minutes.

For the purpose of this patent application, the yield (of ARA-containing oil) of comparative experiment A is defined as 100%. The yields obtained in the following experiments are given vis-a-vis the yield of comparative experiment A.

### Reference experiment B.

Comparative experiment A was repeated, with the difference that no extrusion or drying was performed. The mixture was extracted using the same procedure as described in comparative experiment A. The yield was 29,68%.

### Example 1

Reference experiment B was repeated, with the difference that 82 weight parts of the squeezed filter cake were crumbled, and mixed with 18 weight parts of silica (SIPERNAT® 22 obtained from Degussa AG, Germany) in a Hobart high shear mixer. Extraction was performed in the same manner. The yield was 84.68%, which is an increase with 55% compared to the reference experiment wherein no desiccant was used.

### Example 2

Example 1 was repeated with the difference that the quantity of silica was increased from 18 weight parts to 25 weight parts. This resulted in a further increase of the yield (with 4.8%) to a value of 89.48%

### Example 3

Example 2 was repeated with the difference that the quantity of silica was further increased from 25 weight parts to 35 weight parts. This resulted in a further increase of the yield with 7.53% to a value of 97.01%.

An overview of examples 1,2 and 3 and experiments A and B is given in table 1.
This table shows that the use of a desiccant results in an increase of the extraction yield, and that the extraction yield increases with increasing amount of desiccant used. This enables optimal extraction yields to be obtained by varying the quantity of silica used. The experiments show that virtually the same yield can be obtained by using a desiccant according to the invention, and that extrusion and drying is obviated.

Table 1. Yield obtained by process according to the invention vs. yield of experiments A, B

| Ex. No. | Cake (wt.%) | Silica (wt.%) | yield |
|---|---|---|---|
| Comp. A | Extrusion and drying | | 100 |
| Ref. B. | 100 | 0 | 29.68 |
| Example 1 | 82 | 18 | 84.68 |
| Example 2 | 75 | 25 | 89.48 |
| Example 3 | 65 | 35 | 97.01 |

The lipid obtained by the process according to the invention and that obtained by the process involving extrusion and drying have virtually the same composition, see table 2.

### Example 4

A squeezed filter cake (*Mortierella alpina*) having a dry matter content of 45.6% was prepared as described in the previous examples. 50 g of this cake was mixed with 5 g of poly-acrylate super absorber (Luquasorb® FP 800, obtained from BASF, Germany). The mixture obtained was extracted with 250 ml of hexane under stirring. The quantity of extracted ARA-containing oil was determined after 7 hours of extraction (after evaporation of the hexane), and was found to be 7.8 g.

### Example 5

Example 4 was repeated with the difference that 10 g of silica desiccant (SIPERNAT® 22 obtained from Degussa AG, Germany) was used instead of 5 g of polyacrylate. After 7 hours of extraction, the quantity of extracted ARA-containing oil was 7.8 g.

Performing the extraction of examples 4 and 5 using dried extrudates as disclosed in WO-A-9736996 (in the absence of desiccant) results in between 7.65 and 8.1 g of extracted oil.

It is clearly seen that by using a desiccant according to the invention the same yield can be obtained without the need for granulation/extrusion and/or drying by evaporation.

Table 2. Composition of lipid (oil) obtained by process according to the invention vs. experiments A,B

| | Invention (as examples 1-3, but with 20 weight parts silica) | Comparative experiment A (extrusion + drying) |
|---|---|---|
| ARA (kg/kg) | 404.5 | 421.5 |
| ARA % of total fatty acids | 46.4 | 46.8 |
| Polymers | 0.6 | 0.5 |
| Tri-glycerides | 91.9 | 92.2 |
| di-glycerides | 5.5 | 5.4 |
| sterols | 2 | 2 |
| C14:0 | 0.6 | 0.6 |
| C16:0 | 13.1 | 13.1 |

(continued)

|  | Invention (as examples 1-3, but with 20 weight parts silica) | Comparative experiment A (extrusion + drying) |
|---|---|---|
| C 17:0 | 0.4 | 0.4 |
| C 18:0 | 9.3 | 9.4 |
| C 18:1 w9c | 8.5 | 8.4 |
| C 18:1 w7c | 0.5 | 0.4 |
| C 18:2w6 | 7.6 | 7.4 |
| C20:0 | 0.9 | 0.9 |
| C18:3w6 | 3.9 | 3.8 |
| C20:1w9 | 0.3 | 0.3 |
| C20:2 | 0.7 | 0.7 |
| C22:2 | 1.6 | 1.6 |
| C20:3w6 | 4.7 | 4.7 |
| C24:0 | 1.5 | 1.6 |

**Claims**

1. Process for obtaining lipid from a composition comprising cells and water, said process comprising:

   (a) contacting the composition with a desiccant; and
   (b) recovering the lipid from the cells.

2. Process according to claim 1, wherein the desiccant has an absorption capacity of at least 50 g of water per 100 g of desiccant.

3. Process according to claim 1 or claim 2, wherein the desiccant is porous.

4. Process according to any preceding claim, wherein the desiccant has a specific surface area of at least 25 $m^2/g$.

5. Process according to any preceding claim, wherein the desiccant is selected from silica, activated carbon, activated alumina, a molecular sieve (e.g. a carbon molecular sieve or a zeolite), or a cross-linked polymer (e.g a polyacrylate).

6. Process according to any preceding claim, wherein the desiccant is silica.

7. Process according to any preceding claim, wherein

$$w_{des}/(w_{cells(a)} + w_{w(a)}) > (1/x)^*(1 - w_{cells(a)}/(0.8^*(w_{cells(a)} + w_{w(a)}))),$$

wherein
$w_{des}$ = weight of the desiccant to be contacted with the composition in (a)
$w_{cells(a)}$ = weight of dry matter of the cells in the composition to be contacted with the desiccant in (a)
$w_{w(a)}$ = weight of the water in the composition to be contacted with the desiccant in (a)
x = absorption capacity of the desiccant.

8. Process according to any preceding claim, wherein the process comprises obtaining the cells by fermentation.

9. Process according to any preceding claim, wherein the composition to be contacted with the desiccant in (a) is a fermentation broth or wherein the composition to be contacted with the desiccant in (a) is obtained by separating water from a fermentation broth, for instance by mechanical separation.

**10.** Process according to any preceding claim, wherein the composition to be contacted with the desiccant in (a) has a water content of between 5 and 95 wt. % .

**11.** Process according to any preceding claim, wherein (a) comprises mixing the composition with the desiccant to give a mixture comprising cells and desiccant; and wherein (b) comprises recovering the lipid from the mixture, optionally in the presence of at least part of the desiccant.

**12.** Process according to claim 11, wherein said process comprises at least one of the following:

(i) separating at least part of the desiccant from the mixture; and/or
(ii) $w_{w(b)}w_{cells(b)} > 0.5*w_{w(a)}/w_{cells(a)}$, wherein

$w_{w(b)}$ = weight of water in mixture from which the lipid is recovered in (b)
$w_{cells(b)}$ = weight of dry matter of the cells in mixture from which the lipid is recovered in (b)
$w_{w(a)}$ = weight of water in the composition to be contacted with the desiccant in (a)
$w_{cells(a)}$ = weight of dry matter of the cells in the composition to be contacted with the desiccant in (a)

**13.** Process according to any preceding claim, wherein (b) comprises recovering the lipid by extraction with a solvent.

**14.** Process according to any preceding claim, wherein the solvent is an apolar solvent, for instance a $C_{3-6}$ alkane, preferably hexane.

**15.** Process according to any preceding claim, wherein the solvent a supercritical fluid, for instance liquid $CO_2$ or supercritical propane.

**16.** Process according to any preceding claim, wherein said process does not comprise, after (a) and prior to (b):

a drying step that involves heating of the cells; and/or
granulating the cells to obtain granular particles.

**17.** Process according to any preceding claim, wherein the cells are microbial cells, for instance yeast cells, bacterial cells, fungal cells, or algal cells.

**18.** Process according to any preceding claim, wherein the cells are from the genus *Mortierella* or from *Pythium* or *Entomophthora.*

**19.** Process according to any preceding claim, wherein the cells are from the genus *Crypthecodinium,* or of the order Thraustochytriales, for instance from the genus *Thraustochytrium or Schizochytrium.*

**20.** Process according to any preceding claim, wherein the cells are plant cells, for instance cells from seeds or nuts.

**21.** Process according to any preceding claim, wherein said lipid comprises a polyunsaturated fatty acid (PUFA), for instance an Ω-3 PUFA or an Ω-6 PUFA.

**22.** Process according to any preceding claim, wherein said PUFA is a PUFA having at least 18 carbon atoms.

**23.** Process according to any preceding claim, wherein said PUFA is arachidonic acid (ARA).

**24.** Process according to any preceding claim, wherein said PUFA is docosahexaenoic acid (DHA).

**25.** Process according to any preceding claim, wherein said lipid comprises at least 5 wt.% of said PUFA.

**26.** Process according to any preceding claim, wherein said lipid comprises a one or more of the following compounds: lipstatin, statin, TAPS, pimaricine, nystatine, fat-soluble antibiotic (e.g. laidlomycin) fat-soluble anti-oxidant (e.g. co-enzyme Q10), cholesterol, phytosterol, desmosterol, tocotrenol, tocopherol, carotenoid, or xanthopphylls, for instance beta-carotene, lutein, lycopene, astaxanthin, zeaxanthin, or canthaxanthin.

**27.** Mixture comprising (i) cells comprising a lipid and (ii) a desiccant, wherein said lipid is a lipid as defined in any one of claims 21 to 26, and wherein the cells are cells as defined in any one of claims 17 to 19.

**28.** Mixture according to claim 27, wherein said desiccant is a desiccant as defined in any one of claims 2 to 6.

**29.** Mixture according to claim 27 or 28, said mixture further comprising water, wherein

$$W_{des,\ mix}/(W_{cells,\ mix} + W_{w,\ mix}) > (1/x)*(1 - W_{cells,\ mix}/(0.8*(W_{cells,\ mix} + W_{w,\ mix}))),$$

preferably $W_{des,\ mix}/(W_{cells,\ mix} + W_{w,\ mix}) > (1/x)*(1 - W_{cells,\ mix}/(0.9*(W_{cells,\ mix} + W_{w,\ mix})))$, wherein
$W_{des,\ mix}$ = weight of the desiccant in the mixture
$W_{cells\ mix}$ = weight of dry matter of the cells in the mixture
$W_{w,\ mix}$ = weight of the water in mixture
$x$ = absorption capacity of the desiccant.

**30.** Process for obtaining a lipid, said process comprising contacting a mixture according to any one of claims 27 to 29 with an organic solvent.

**Patentansprüche**

**1.** Verfahren zum Erhalt von Lipid aus einer Zusammensetzung, die Zellen und Wasser umfaßt, bei dem man:

(a) die Zusammensetzung mit einem Trockenmittel in Berührung bringt und
(b) das Lipid aus den Zellen zurückgewinnt.

**2.** Verfahren nach Anspruch 1, bei dem das Trockenmittel eine Absorptionskapazität von mindestens 50 g Wasser pro 100 g Trockenmittel aufweist.

**3.** Verfahren nach Anspruch 1 oder Anspruch 2, bei dem das Trockenmittel porös ist.

**4.** Verfahren nach einem der vorhergehenden Ansprüche, bei dem das Trockenmittel eine spezifische Oberfläche von mindestens 25 m²/g aufweist.

**5.** Verfahren nach einem der vorhergehenden Ansprüche, bei dem man das Trockenmittel unter Siliciumdioxid, Aktivkohle, aktiviertem Aluminiumoxid, einem Molsieb (z.B. einem Kohlenstoff-Molsieb oder einem Zeolith) oder einem vernetzten Polymer (z.B. einem Polyacrylat) auswählt.

**6.** Verfahren nach einem der vorhergehenden Ansprüche, bei dem es sich bei dem Trockenmittel um Siliciumdioxid handelt.

**7.** Verfahren nach einem der vorhergehenden Ansprüche, bei dem

$$W_{Tro}/(W_{Zellen(a)} + W_{W(a)}) > (1/x)*(1 - W_{Zellen(a)}/(0,8*(W_{Zellen(a)} + W_{W(a)}))),\ worin$$

$W_{Tro}$ = Gewicht des in (a) mit der Zusammensetzung in Berührung zu bringenden Trockenmittels
$W_{Zellen(a)}$ = Trockensubstanzgewicht der Zellen in der in (a) mit dem Trockenmittel in Berührung zu bringenden Zusammensetzung
$W_{W(a)}$ = Gewicht des Wassers in der in (a) mit dem Trockenmittel in Berührung zu bringenden Zusammensetzung
$x$ = Absorptionskapazität des Trockenmittels.

**8.** Verfahren nach einem der vorhergehenden Ansprüche, bei dem man die Zellen durch Fermentation erhält.

**9.** Verfahren nach einem der vorhergehenden Ansprüche, bei dem es sich bei der in (a) mit dem Trockenmittel in Berührung zu bringenden Zusammensetzung um eine Fermentationsbrühe handelt oder man die in (a) mit dem Trockenmittel in Berührung zu bringende Zusammensetzung durch Abtrennung von Wasser aus einer Fermentationsbrühe, zum Beispiel durch mechanische Abtrennung, erhält.

**10.** Verfahren nach einem der vorhergehenden Ansprüche, bei dem die in (a) mit dem Trockenmittel in Berührung zu bringende Zusammensetzung einen Wassergehalt zwischen 5 und 95 Gew.-% aufweist.

**11.** Verfahren nach einem der vorhergehenden Ansprüche, bei dem (a) das Mischen der Zusammensetzung mit dem Trockenmittel zur Bildung einer Mischung, die Zellen und Trockenmittel umfaßt, umfaßt und (b) die Gewinnung des Lipids aus der Mischung, gegebenenfalls in Gegenwart von mindestens einem Teil des Trockenmittels, umfaßt.

**12.** Verfahren nach Anspruch 11, bei dem das Verfahren

(i) das Abtrennen mindestens eines Teils des Tockenmittels von der Mischung und/oder
(ii)

$$W_{W(b)}/W_{Zellen(b)} > 0,5*W_{W(a)}/W_{Zellen(a)},$$

worin
$W_{W(b)}$ = Gewicht von Wasser in der Mischung, aus der das Lipid in (b) gewonnen wird,
$W_{Zellen(b)}$ = Trockensubstanzgewicht der Zellen in der Mischung, aus der das Lipid in (b) gewonnen wird,
$W_{W(a)}$ = Gewicht des Wassers in der in (a) mit dem Trockenmittel in Berührung zu bringenden Zusammensetzung
$W_{Zellen(a)}$ = Trockensubstanzgewicht der Zellen in der in (a) mit dem Trockenmittel in Berührung zu bringenden Zusammensetzung
umfaßt.

**13.** Verfahren nach einem der vorhergehenden Ansprüche, bei dem (b) die Gewinnung des Lipids durch Extraktion mit einem Lösungsmittel umfaßt.

**14.** Verfahren nach einem der vorhergehenden Ansprüche, bei dem es sich bei dem Lösungsmittel um ein unpolares Lösungsmittel, zum Beispiel ein $C_{3-6}$-Alkan, vorzugsweise Hexan, handelt.

**15.** Verfahren nach einem der vorhergehenden Ansprüche, bei dem es sich bei dem Lösungsmittel um ein überkritisches Fluid, zum Beispiel flüssiges $CO_2$ oder überkritisches Propan, handelt.

**16.** Verfahren nach einem der vorhergehenden Ansprüche, bei dem das Verfahren nach (a) und vor (b)
keinen Trocknungsschritt, bei dem die Zellen erhitzt werden, und/oder
keine Granulierung der Zellen zum Erhalten von Granulatteilchen
umfaßt.

**17.** Verfahren nach einem der vorhergehenden Ansprüche, bei dem es sich bei den Zellen um Mikrobenzellen, zum Beispiel Hefezellen, Bakterienzellen, Pilzzellen oder Algenzellen, handelt.

**18.** Verfahren nach einem der vorhergehenden Ansprüche, bei dem die Zellen aus der Gattung *Mortierella* oder *Pythium* oder *Entomophthora* stammen.

**19.** Verfahren nach einem der vorhergehenden Ansprüche, bei dem die Zellen aus der Gattung *Crypthecodinium* oder der Ordnung Thraustochytriales, zum Beispiel aus der Gattung *Thraustochytrium* oder *Schizochytrium,* stammen.

**20.** Verfahren nach einem der vorhergehenden Ansprüche, bei dem es sich bei den Zellen um Pflanzenzellen, zum Beispiel Zellen aus Samen oder Nüssen, handelt.

**21.** Verfahren nach einem der vorhergehenden Ansprüche, bei dem das Lipid eine mehrfach ungesättigte Fettsäure (PUFA), zum Beispiel eine Ω-3-PUFA. oder eine Ω-6-PUFA, handelt.

**22.** Verfahren nach einem der vorhergehenden Ansprüche, bei dem es sich bei der PUFA um eine PUFA mit mindestens 18 Kohlenstoffatomen handelt.

**23.** Verfahren nach einem der vorhergehenden Ansprüche, bei dem es sich bei der PUFA um Arachidonsäure (ARA) handelt.

**24.** Verfahren nach einem der vorhergehenden Ansprüche, bei dem es sich bei der PUFA um Docosahexaensäure (DHA) handelt.

**25.** Verfahren nach einem der vorhergehenden Ansprüche, bei dem das Lipid mindestens 5 Gew.-% der PUFA umfaßt.

**26.** Verfahren nach einem der vorhergehenden Ansprüche, bei dem das Lipid eine oder mehrere der folgenden Verbindungen umfaßt: Lipstatin, Statin, TAPS, Pimaricin, Nystatin, fettlösliches Antibiotikum (z.B. Laidlomycin), fettlösliches Antioxidans (z.B. Coenzym Q10), Cholesterol, Phytosterol, Desmosterol, Tocotrenol, Tocopherol, Carotenoid oder Xanthophylle, zum Beispiel beta-Carotin, Lutein, Lycopen, Astaxanthin, Zeaxanthin oder Canthaxanthin.

**27.** Mischung, umfassend (i) Zellen, die ein Lipid umfassen, und (ii) ein Trockenmittel, wobei es sich bei dem Lipid um ein Lipid gemäß einem der Ansprüche 21 bis 26 handelt und es sich bei den Zellen um Zellen gemäß einem der Ansprüche 17 bis 19 handelt.

**28.** Mischung nach Anspruch 27, wobei es sich bei dem Trockenmittel um ein Trockenmitel gemäß einem der Ansprüche 2 bis 6 handelt.

**29.** Mischung nach Anspruch 27 oder 28, die ferner Wasser umfaßt, wobei

$$W_{Tro,Mis} / (w_{Zellen,Mis} + w_{W,Mis}) > (1/x) * (1 - w_{Zellen,Mis} / (0,8 * (W_{Zellen,Mis} + w_{W,Mis}))),$$

vorzugsweise $W_{Tro,Mis} / (w_{zellen, Miss} + w_{w,Mis}) > (1/x) * (1 - w_{zellen,Mis} / (0,9 * (w_{zellen,Mis} + w_{w,Mis})))$, worin
$w_{Tro,Mis}$ = Gewicht des Trockenmittels in der Mischung
$W_{zellen,Mis}$ = Trockensubstanzgewicht der Zellen in der Mischung
$w_{w,Mis}$ = Gewicht des Wassers in der Mischung
$x$ = Absorptionskapazität des Trockenmittels.

**30.** Verfahren zum Erhalt eines Lipids, bei dem man eine Mischung nach einem der Ansprüche 27 bis 29 mit einem organischen Lösungsmittel in Berührung bringt.

**Revendications**

**1.** Procédé d'obtention de lipides à partir d'une composition comprenant des cellules et de l'eau, ledit procédé comprenant :

(a) la mise en contact de la composition avec un agent déshydratant ; et
(b) la récupération des lipides à partir des cellules.

**2.** Procédé selon la revendication 1, dans lequel l'agent déshydratant a une capacité d'absorption d'au moins 50 g d'eau pour 100 g d'agent déshydratant.

**3.** Procédé selon la revendication 1 ou 2, dans lequel l'agent déshydratant est poreux.

**4.** Procédé selon l'une quelconque des revendications précédentes, dans lequel l'agent déshydratant possède une surface spécifique d'au moins 25 m$^2$/g.

**5.** Procédé selon l'une quelconque des revendications précédentes, dans lequel l'agent déshydratant est choisi parmi la silice, le charbon actif, l'alumine activée, un tamis moléculaire (par exemple un tamis moléculaire carboné ou une zéolithe) ou un polymère réticulé (par exemple un polyacrylate).

**6.** Procédé selon l'une quelconque des revendications précédentes, dans lequel l'agent déshydratant est la silice.

**7.** Procédé selon l'une quelconque des revendications précédentes, dans lequel

$$W_{des}/(W_{cells(a)} + W_{w(a)}) > (1/X)*(1-W_{cells(a)}/(0,8*(W_{cells(a)} + W_{w(a)}))), \text{ où}$$

$W_{des}$ = poids de l'agent déshydratant à mettre en contact avec la composition dans (a),
$W_{cells(a)}$ = poids de matière sèche des cellules dans la composition à mettre en contact avec l'agent déshydratant dans (a),
$w_{w(a)}$ = poids de l'eau dans la composition à mettre en contact avec l'agent déshydratant dans (a),
$X$ = capacité d'absorption de l'agent déshydratant.

**8.** Procédé selon l'une quelconque des revendications précédentes, dans lequel le procédé comprend l'obtention des cellules par fermentation.

**9.** Procédé selon l'une quelconque des revendications précédentes, dans lequel la composition à mettre en contact avec l'agent déshydratant dans (a) est un bouillon de fermentation ou dans lequel la composition à mettre en contact avec l'agent déshydratant dans (a) est obtenue par séparation de l'eau à partir d'un bouillon de fermentation, par exemple par séparation mécanique.

**10.** Procédé selon l'une quelconque des revendications précédentes, dans lequel la composition à mettre en contact avec l'agent déshydratant dans (a) possède une teneur en eau comprise entre 5 et 95% en poids.

**11.** Procédé selon l'une quelconque des revendications précédentes, dans lequel (a) comprend le mélange de la composition avec l'agent déshydratant pour fournir un mélange comprenant des cellules et de l'agent déshydratant ; et dans lequel (b) comprend la récupération des lipides à partir du mélange, éventuellement en présence d'au moins une partie de l'agent déshydratant.

**12.** Procédé selon la revendication 11, dans lequel ledit procédé comprend au moins un parmi

(i) la séparation d'au moins une partie de l'agent déshydratant à partir du mélange ; et/ou
(ii)

$$W_{w(b)}/W_{cells(b)} > 0,5*W_{w(a)}/W_{cells(a)},$$

où
$W_{w(b)}$ = poids de l'eau dans le mélange à partir duquel les lipides sont récupérés dans (b),
$W_{cells(b)}$ = poids de matière sèche des cellules dans le mélange à partir duquel les lipides sont récupérés dans (b),
$W_{w(a)}$ = poids de l'eau dans la composition à mettre en contact avec l'agent déshydratant dans (a),
$W_{cells(a)}$ = poids de matière sèche des cellules dans la composition à mettre en contact avec l'agent déshydratant dans (a).

**13.** Procédé selon l'une quelconque des revendications précédentes, dans lequel (b) comprend la récupération des lipides par extraction à l'aide d'un solvant.

**14.** Procédé selon l'une quelconque des revendications précédentes, dans lequel le solvant est un solvant apolaire, par exemple un alcane en $C_{3-6}$, préférablement l'hexane.

**15.** Procédé selon l'une quelconque des revendications précédentes, dans lequel le solvant est un fluide supercritique, par exemple du $CO_2$ liquide ou du propane supercritique.

**16.** Procédé selon l'une quelconque des revendications précédentes, dans lequel ledit procédé ne comprend pas, après (a) et avant (b) :

une étape de séchage impliquant le chauffage des cellules ; et/ou
la granulation des cellules afin d'obtenir des particules granulaires.

**17.** Procédé selon l'une quelconque des revendications précédentes, dans lequel les cellules sont des cellules microbiennes, par exemple des cellules de levure, des cellules bactériennes, des cellules fongiques ou des cellules algales.

**18.** Procédé selon l'une quelconque des revendications précédentes, dans lequel les cellules sont issues du genre *Mortierella* ou de *Pythium* ou *Entomophthora.*

**19.** Procédé selon l'une quelconque des revendications précédentes, dans lequel les cellules sont issues du genre *Crypthecodinium,* ou de l'ordre Thraustochytriales, par exemple du genre *Thraustochytrium* ou *Schizochytrium.*

**20.** Procédé selon l'une quelconque des revendications précédentes, dans lequel les cellules sont des cellules végétales, par exemple des cellules issues de graines ou de noix.

**21.** Procédé selon l'une quelconque des revendications précédentes, dans lequel lesdits lipides comprennent un acide gras polyinsaturé (AGPI), par exemple un AGPI Ω-3 ou un AGPI Ω-6.

**22.** Procédé selon l'une quelconque des revendications précédentes, dans lequel ledit AGPI est un AGPI ayant au moins 18 atomes de carbone.

**23.** Procédé selon l'une quelconque des revendications précédentes, dans lequel ledit AGPI est l'acide arachidonique (ARA).

**24.** Procédé selon l'une quelconque des revendications précédentes, dans lequel ledit AGPI est 1"acide docosahexaénoïque (DHA).

**25.** Procédé selon l'une quelconque des revendications précédentes, dans lequel lesdits lipides comprennent au moins 5% en poids dudit AGPI.

**26.** Procédé selon l'une quelconque des revendications précédentes, dans lequel lesdits lipides comprennent un ou plusieurs parmi les composants suivants : la lipstatine, la statine, le TAP, la pimaricine, la nystatine, des antibiotiques liposolubles (par exemple la laidlomycine), des antioxydants liposolubles (par exemple le coenzyme Q10), le cholestérol, le phytostérol, le desmostérol, le tocotrénol, le tocophérol, les caroténoïdes, ou les xanthophylles, par exemple le bêta-carotène, la lutéine, le lycopène, l'astaxanthine, la zéaxanthine ou la canthaxanthine.

**27.** Mélange comprenant (i) des cellules comprenant des lipides et (ii) un agent déshydratant, dans lequel lesdits lipides sont des lipides tels que définis selon l'une quelconque des revendications 21 à 26, et dans lequel les cellules sont des cellules telles que définies selon l'une quelconque des revendications 17 à 19.

**28.** Mélange selon la revendication 27, dans lequel ledit agent déshydratant est un agent déshydratant tel que défini selon l'une quelconque des revendications 2 à 6.

**29.** Mélange selon la revendication 27 ou 28, ledit mélange comprenant en outre de l'eau, dans lequel
$W_{des,\ mix}/(W_{cells,\ mix} + W_{w,\ mix}) > (1/x) * (1 - W_{cells,\ mix}/(0,8*(W_{cells,\ mix} + Ww, mix)))$, préférablement $W_{des,\ mix}/(W_{cells,\ mix} + Ww, mix) > (1/x) * (1 - W_{cells,\ mix}/ (0, 9* (Wcells, mix\ W_{w,}\ mit)))$, Où
$W_{des,\ mix}$ = poids de l'agent déshydratant dans le mélange,
$W_{cells,\ mix}$ = poids de matière sèche des cellules dans le mélange,
$W_{w,\ mix}$ = poids de l'eau dans le mélange
X = capacité d'absorption de l'agent déshydratant.

**30.** Procédé d'obtention de lipides, ledit procédé comprenant la mise en contact d'un mélange selon l'une quelconque des revendications 27 à 29 avec un solvant organique.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9736996 A
- WO 0077183 A
- WO 9737032 A